(19) Europäisches Patentamt / European Patent Office / Office européen des brevets

(11) **EP 2 353 579 B1**

(12) **EUROPEAN PATENT SPECIFICATION**

(45) Date of publication and mention
of the grant of the patent:
**05.12.2018 Bulletin 2018/49**

(51) Int Cl.:
*A61K 8/34* $^{(2006.01)}$  *A61Q 15/00* $^{(2006.01)}$
*A61Q 17/00* $^{(2006.01)}$

(21) Application number: **10152998.0**

(22) Date of filing: **09.02.2010**

(54) **Deodorant composition**

**Desodorierende Zusammensetzung**

**Composition déodorante**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**10.08.2011 Bulletin 2011/32**

(73) Proprietor: **Colgate-Palmolive Europe SARL Geneva 1204 (CH)**

(72) Inventors:
- **de Beer, Andrew Iwan**
**2729 CK Zoetermeer (NL)**
- **Buitelaar, Thomas Andreas**
**3155 VC Maasland (NL)**
- **Olierook, Marco Johannes Victor**
**2718 KA Zoetermeer (NL)**

(74) Representative: **Wichmann, Hendrik et al Wuesthoff & Wuesthoff Patentanwälte PartG mbB Schweigerstraße 2 81541 München (DE)**

(56) References cited:
**EP-A1- 1 604 642       WO-A1-01/85130
WO-A2-91/13606      WO-A2-2008/114189
DE-A1- 1 961 152       DE-A1- 2 531 343
DE-A1- 10 137 901      US-A1- 2009 317 345**

- **A. KLUGE, D. KRUSE: "Eigenschaften und Verwendung eines neuen C16-Alkohols" SEIFEN - ÖLE - FETTE - WACHSE, vol. 97, no. 22, 1971, pages 827-831, XP9137346**
- **DATABASE STN CHEMICAL ABSTRACTS, X [Online] 27 October 1980 (1980-10-27), KATO ET AL: "The antimicrobial characteristics of 1-alkanols" XP002074350 retrieved from CHEMICAL**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The invention relates to compositions that serve the purpose of inhibiting the odor components that result from human sweat (in brief: a deodorant composition). More particularly, the invention pertains to deodorant compositions that have a bactericidal effect.

### Background of the invention

It is customary for many people to apply cosmetic compositions to their armpits and the like in order to prevent the excretion of sweat or the undesired (odor) effects thereof.

The prevention of the secretion of sweat generally occurs by antiperspirants. Examples hereof include widely used aluminum and/or zirconium compounds and complexes, such as aluminum chloride, aluminum chlorohydrate, zirconyl hydroxychloride salts, aluminum zirconium glycine complexes, see e.g. EP 295 071, but also polyols as described in e.g. EP 16 04 642, or short-chain vicinal diols as described in WO 2009/07089. Antiperspirants serve to form a plug in the ducts of the sweat glands.

The use of antiperspirants, although generally effective, is not desirable for all people in all circumstances. Particularly, sweating serves physiological purposes, the most important one of which is thermoregulation. Nevertheless, in many social circumstances sweating is undesired, and certainly the odor of sweat is not socially acceptable.

The prevention of the odor of sweat generally occurs by deodorants. In general, active agents in deodorants serve to kill bacteria or limit the growth thereof. These agents are generally referred to as antimicrobial, antibacterial, or bactericide agents. The general theory behind such antimicrobial action is that particularly bacteria cause the otherwise neutrally smelling sweat to assume an unpleasant and/or unacceptable odor.

The present invention is in the field of deodorant compositions. I.e., the invention expressly does not relate to preventing the secretion of sweat itself, but of the undesired odorous effects thereof.

Deodorants generally comprise bactericidal active substances such as triclosan and other, typically chlorinated bactericides. A drawback to the existing bactericides is that they can cause skin irritation, are considered hazardous chemicals, and their widespread use has lead to concerns of bacteria developing resistance against these agents.

Hence, a desire exists for new agents that serve to combat sweat malodor, and particularly *in lieu* of classical bactericides.

A recent reference on deodorants is WO 2009/046008 , which relates to propylene glycol / glycerin-based deodorants. Disclosed is a deodorant base composition comprising a formulation of propylene glycol, glycerin, and water. This base composition itself, expressly, is not a deodorant. It is indicated that to this base composition deodorancy agents can be added, such as antimicrobials and/or nature-based deodorizing agents such as hops extracts or zinc ricinoleate. In addition to propylene glycol, the reference also mentions other polyhydric alcohols having 2 to 6 carbon atoms and 2 to 6 hydroxyl groups, e.g. ethylene glycol and 1,3-propane diol, a function of which is not indicated in the document.

A. Kluge et al in "Eigenschaften und Verwendung eines neuen C16-Alkohols" Seifen - Ole - Fette - Wachse, Vol. 97, No. 22, 1971, pages 827-831 discloses the use of 2-hexyl decanol in cosmetics. DE 25 31 343 A2 discloses antimicrobial compositions containing substituted N-benzoyl-N'phenyl-guanidines as active agents. DE 101 37 901 A1 and WO 2008/114189 A2 disclose compositions containing alcohols and carriers. DE 19 61 152 A1 discloses bactericidal and fungicidal skin preparations for external application containing an aliphatic, saturated or unsaturated branched chain alcohol. WO 91/13606 A2 discloses the use of 3,7-dimethyl-octane-1-ol as an antimicrobial active agent. US 2009/317345 A1 discloses deodorant compositions comprising dipropylene glycol and octyldodecanol. WO 01/85130 A1 discloses an aerosol deodorant composition comprising dipropylene glycol and a mixture of proptane/butane as propellant.

### Summary of the Invention

The use of a composition as a deodorant on human skin according to the present invention is defined in the claims.

In order to better address one or more of the foregoing desires in one aspect useful for understanding the present invention, a deodorant composition is provided that comprises a carrier selected from the group consisting of monohydric and polyhydric alcohols having an octanol/water partition coefficient lower than that of octanol and a deodorant active agent selected from the group of monohydric and polyhydric alcohols having a an octanol/water partition coefficient higher than that of octanol.

In another aspectuseful for understanding the invention, presented is the use of an alcohol selected from the group consisting of monohydric and polyhydric alcohols having an octanol/water partition coefficient lower than that of octanol, as a carrier for a deodorant active agent selected from the group of monohydric and polyhydric alcohols having an octanol/water partition coefficient higher than that of octanol, for the purpose of activating the active agent on human skin in the presence of sweat.

In a further aspect, useful for understanding the invention, presented is the use of an alcohol selected from the group consisting of monohydric and polyhydric alcohols having an octanol/water partition coefficient higher than that of octanol, as an antimicrobials active agent in a deodorant product.

In yet another aspectuseful for understanding the invention, presented is a cosmetic method for reducing human body odor, the method comprising applying on a body part prone to the excretion of sweat a composition comprising a deodorant active agent selected from the group of monohydric and polyhydric alcohols having an octanol/water partition coefficient higher than that of octanol, wherein the composition comprises a carrier selected from the group consisting of monohydric and polyhydric alcohols having an octanol/water partition coefficient lower than that of octanol.

In a still further aspectuseful for understanding the invention, presented is a method for reducing bacterial activity in human sweat, the method comprising applying on a body part prone to the excretion of sweat a composition comprising a deodorant active agent selected from the group of monohydric and polyhydric alcohols having an octanol/water partition coefficient higher than that of octanol, wherein the composition comprises a carrier selected from the group consisting of monohydric and polyhydric alcohols having an octanol/water partition coefficient lower than that of octanol.

Detailed Description of the Invention

The invention in a broad sense is based on the recognition that the process of the excretion of sweat changes the environment on the skin, in the sense that the otherwise lipid skin surface will in effect become an aqueous environment. The invention acknowledges that an effective deodorant can be formulated if use is made of this phenomenon.

The invention makes use of deodorant active ingredients that, by their relatively lipid nature, would normally tend to penetrate into the skin. Thus, to the extent that these compounds would normally exert any effect after application onto the skin, such an effect would be based on action inside the skin, but they are not themselves sufficiently active in the aqueous environment caused by the excretion of sweat. Thus, e.g., some of the deodorant active ingredients suitable for use in the present invention, may as such display a different function, e.g. as an antiperspirant.

By virtue of the present invention, the active is not only rendered active in the environment of sweat on the skin, but at the same time is largely prevented from exerting unwanted action within the skin.

In a preferred embodiment, the components of the composition of the invention are such that they form a one-phase system in the environment of human skin. The invention, by virtue of the selection of polyol-type carriers of a less lipophilic character, makes it possible to thus have a desirable one phase composition applied on the skin, whilst rendering the deodorant active agent in an active mode when sweat is present.

The deodorant active agent is, for purposes of understanding the present invention, generally selected from the group consisting of monohydric and polyhydric alcohols having an octanol/water partition coefficient higher than that of octanol.

More precisely phrased, the partition coefficient $P_{oct/wat}$ reflects the partition of a substance, in this case a deodorant active monohydric or polyhydric alcohol, between the two phases of water and octanol. This refers to the following formula:

$$\log P_{oct/wat} = \log \left( \frac{[solute]_{octanol}}{[solute]_{water}^{un-ionised}} \right)$$

The absolute values for the partition coefficient Poct/wat will depend on temperature and pH, whereby a standard method in the art, applied in the present application, is to conduct the measurement at 25°. Where the octanol/water partition coefficient of a substance in water is below that of octanol, this means that the partition or distribution of the substance is more in water, and less in octanol, than the water/octanol system. Put in different wording, this will be recognized as a substance that is less lipophilic, and more hydrophilic than the reference substance octanol. Vice versa, where the octanol/water partition coefficient of a substance in water is higher than that of octanol, this means that the partition or distribution of the substance is more in octanol and less in water, than the water/octanol system. Put in different wording, this will be recognized as a substance that is more lipophilic, and less hydrophilic than the reference substance octanol.

In general, the deodorant active monohydric and polyhydric alcohols useful for understanding the invention have a log $P_{oct/wat}$ of greater than 2, more preferably 4 or more (e.g. 2-butyl octanol), and most preferably of from 6 (e.g. 2-hexyl decanol) to 8 (e.g. 2-octyl dodecanol). In one deodorant composition, combinations of different deodorant active alcohols can be applied.

Whilst the active agents are described hereinbefore as deodorant active agents, it is preferred that the deodorant activity is in fact based on anti-bacterial activity. The preferred class of deodorant active agents for use in the invention is therefore comprised of anti-bacterially active alcohols, preferably bactericidal active alcohols.

Preferred deodorant active alcohols useful for understanding the invention are linear or branched $C_8$ to $C_{24}$ alcohols (i.e. the molecule having of from eight to twenty-four carbon atoms), preferably $C_{12}$ to $C_{20}$ alcohols.

More preferably, the longest chain present in the molecule is a $C_8$ to $C_{16}$ chain, more preferably $C_{10}$ to $C_{12}$. Most preferably, the alcohol molecule has a $C_{10}$ to $C_{14}$ chain and a $C_4$ to $C_8$ branch, most preferably a $C_{12}$ chain and a $C_8$ branch.

The alcohols can comprise terminal hydroxyl groups, such as fatty alcohols, e.g. $C_n$-OH, with n being an integer of from 6 to 16, preferably $C_8$ to $C_{12}$ alcohols. The alcohols can also be guerbet alcohols comprising secondary hydroxyl groups, e.g. $C_p$-(OH)-$C_q$, with p and q being integers, p being 6 to 16, preferably 10 to 12, and q being 4 to 10, preferably 6-8, e.g. $C_{12}$-(OH)-$C_6$. The alcohols can also be diols, preferably 1,2 vicinal diols having a chain length of from $C_5$ to $C_{14}$.

Without wishing to be bound by theory, the inventors believe that the active alcohols exhibit antimicrobial action by destroying the bacterial cell membrane.

Preferred active agents useful for understanding the invention are monohydric alcohols, particularly the aforementioned guerbet alcohols, as they are substantially odorless themselves.

The active agents for use in the present invention are selected from the group consisting of is 2-hexyl decanol, 2 octyl dodecanol, and combinations thereof.

The carrier is generally selected from the group consisting of monohydric or polyhydric alcohols having an octanol/water partition coefficient lower than that of octanol. In general, the deodorant active monohydric and polyhydric alcohols useful for understanding the invention have a log $P_{oct/wat}$ of below 2, more preferably 0 or lower (e.g. 1,5 pentane diol or 2-methyl 1,3-propane diol), and most preferably of from -1 to -0.5 (e.g. propylene glycol or dipropylene glycol).

Preferred as carriers are polyhydric alcohols having a chain length of $C_2$-$C_6$ and 2-3 hydroxyl groups. More preferably, the carrier is selected from polyhydric alcohols having a chain length of $C_2$-$C_4$ and comprising 2 hydroxyl groups. A most preferred carrier is propylene glycol (log $P_{oct/wat}$ of approximately -1).

The carbon chain of the carrier can be interrupted by one or more oxygen atoms (so as to result in an ether). A carrier of this type used in the invention is dipropylene glycol (i.e. an ether having two hydroxyl groups), having a log $P_{oct/wat}$ of approximately -0.5.

The compositions of the invention can comprise the active alcohol and the carrier in a wide range of weight ratio's. Generally the composition, with the balance being water, will comprise of from 10 wt.% to 50 wt.%, preferably 20 wt% to 30 wt.%, of the carrier and of from 2 wt.% to 20 wt.%, preferably 5 wt.% to 10 wt.% of the active alcohol.

By virtue of the provision of a carrier suitable to activate the aforementioned alcohols with high octanol/water partition, the present inventors have acknowledged the advantageous potential of these compounds as deodorant active agents. In this respect it should be noted that these alcohols are not normally recognized as deodorant active agents, let alone as antimicrobial or bactericide active agents.

Therefore, the invention resides in the use of an alcohol selected from the group consisting of monohydric and polyhydric alcohols having a an octanol/water partition coefficient higher than that of octanol, as an antimicrobial active agent in a deodorant product, as further defined in the claims. With reference to preferred alcohols, particularly provided is the use, as an antimicrobial active agent in a deodorant product of linear or branched $C_8$ to $C_{24}$ alcohols (i.e. the molecule having of from eight to twenty-four carbon atoms), preferably $C_{12}$ to $C_{20}$ alcohols. More preferably, the longest chain present in the molecule is a $C_8$ to $C_{16}$ chain, more preferably $C_{10}$ to $C_{12}$. Most preferably, the alcohol molecule has a $C_{10}$ to $C_{14}$ chain and a $C_4$ to $C_8$ branch, most preferably a $C_{12}$ chain and a $C_8$ branch. The invention relates to the use of 2-hexyl decanol, 2 octyl dodecanol, or a combination thereof, as an antimicrobial active agent in a deodorant product.

With reference to the role of the deodorant active alcohol useful for understanding the invention, provided is a cosmetic method for reducing human body odor, the method comprising applying on a body part prone to the excretion of sweat a composition comprising a deodorant active agent selected from the group of monohydric and polyhydric alcohols having an octanol/water partition coefficient higher than that of octanol, wherein the composition comprises a carrier selected from the group consisting of monohydric and polyhydric alcohols having an octanol/water partition coefficient lower than that of octanol.

In a related, though different aspect useful for understanding the invention, provided is a method for reducing bacterial activity in human sweat, the method comprising applying on a body part prone to the excretion of sweat a composition comprising a deodorant active agent selected from the group of monohydric and polyhydric alcohols having an octanol/water partition coefficient higher than that of octanol, wherein the composition comprises a carrier selected from the group consisting of monohydric and polyhydric alcohols having an octanol/water partition coefficient lower than that of octanol.

In these methods, the preferred active alcohols and carriers are those mentioned hereinbefore.

Considering the role of the carrier in the present disclosure, it is to be noted that some of these alcohols, and notably polyols, having a relatively octanol/water partition, have been used in the art in deodorant base compositions. According to the disclosure, a particularly beneficial use has been found, viz. as carriers in deodorant compositions for the purpose of rendering the aforementioned alcohols, i.e. those selected from the group consisting of monohydric and polyhydric alcohols having an octanol/water partition coefficient lower than that of octanol, active on human skin, notably in the presence of sweat. The preferred carriers for this use are those mentioned above.

The compositions according to the disclosure can be used in deodorant products of generally all applicable deodorant formats,

particularly for application to the underarm (armpit) area. Among these formats are aerosol, pump spray, roll-on, deodorant pads and stick applicators.

Stick applicators are essentially made of a solid or semi-solid material (i.e., a base composition that is firm to the touch) impregnated with active ingredients, fragrances, stabilizers, moisturizers, etc. The base composition materials of deodorant sticks may contain gelling agents, such as those selected from high melting point waxes (including beeswax, montan, ozokerite, ceresin, paraffin, synthetic waxes, hydrogenated castor oil); low melting point waxes (including fatty alcohols containing from about 8 - 20 carbons), and silicone waxes. A more particular group of gelling agents includes stearyl alcohol and hydrogenated castor oil. A stick may also comprise solidifying and/or thickening agents, such as crystalline waxes, cetyl stearate, stearyl stearate, cetyl myristate, cetyl palmitate, stearoxydimethicone, and microcrystalline waxes.

Aerosol applicators are preferred. In addition to the aforementioned carrier and active agent, an aerosol formulation will comprise a propellant. The propellant preferably is a gas selected from propane, isobutane, n-butane and dimethyl ether. Other suitable propellants are known in the art, e.g. an inorganic gas or other gaseous organic compound. The propellant concentration in an aerosol of the invention is usually at least 50 wt. %, preferably at least 70 wt. %, in particular at least 75 wt. % based on the weight of the aerosol. The concentration is usually up to 99.5 wt. %, in particular up to 99.0 wt. %, more in particular up to 95 wt. %, based on total weight of the aerosol. The aerosol may be present in any container suitable for holding an aerosol. Such containers are generally known in the art.

Deodorant products comprising the compositions according to the disclosure can comprise other components frequently present in such compositions, such as perfumes and emollients. Such components are known in the art. A general reference on compositions and formats of deodorant products is: "Antiperspirants and Deodorants" volume 20 edited by Karl Laden, of the "Cosmetics science and technology" series . Reference is particularly made to chapter 9 "A guide to understand deodorants formulations."

It is not excluded that the compositions of the disclosure comprise antiperspirants, if such additional action were desired. It will be clear that this is not a preference in the present disclosure. Preferred deodorant compositions of the disclosure do not have a substantial anti-perspirant activity and will, after application to human skin, particularly on areas having apocrine sweat glands, allow said glands to secrete sweat.

It is not excluded that the compositions of the disclosure comprise additional antimicrobial agents, such as those customary in the art. It will be understood that this is not a preference in the present disclosure. In preferred deodorant compositions of the disclosure, the sole deodorizing action, and particularly the sole anti-microbial activity, will be exhibited by the deodorant active alcohols as described hereinbefore.

It is to be understood that the invention is not limited to the embodiments as described hereinbefore. It is also to be understood that in the claims the word "comprising" does not exclude other elements or steps. Where an indefinite or definite article is used when referring to a singular noun e.g. "a" or "an", "the", this includes a plural of that noun unless something else is specifically stated.

The invention will be illustrated with reference to the following, non-limiting Example and the accompanying non-limiting Figures.

Example 1

Test deodorant products were prepared by mixing a liquid concentrate comprising a carrier (dipropyleneglycol) and different deodorant active alcohols according to the table below. The mixed concentrates are filled into a suitable aerosol container. The aerosol container is closed with a suitable valve, and pressurized with a propellant, isobutane.

Table

|  |  | Deo A | Deo B | Deo C |
|---|---|---|---|---|
| Concentrate | Dipropyleneglycol | 20% | 20% | 20% |
|  | Octyldodecanol | 20% |  |  |
|  | Hexyldecanol |  | 20% | 16% |

(continued)

|  |  | Deo A | Deo B | Deo C |
|---|---|---|---|---|
|  | Butyloctanol |  |  | 4% |
| Propellant | Isobutane | 60% | 60% | 60% |

Example 2

The compositions of Example 1 are tested for deodorizing activity as follows.

A group of 33 subjects received the respective compositions (12 received A, 9 received B, and 12 received C). The instruction was to treat, for two weeks at home, one armpit with the composition received, and leave the other armpit untreated.

After this pretreatment at home, a controlled application was conducted at test facilities in the lab. At two points in time, 8 hours and 24 hours, after this controlled application, the subjects performed a malodor self-assessment, comparing treated vs. untreated armpits, and rated the malodor on a scale of from 1 to 6, a higher score meaning a stronger malodor.

The results of the test are depicted in the figures.

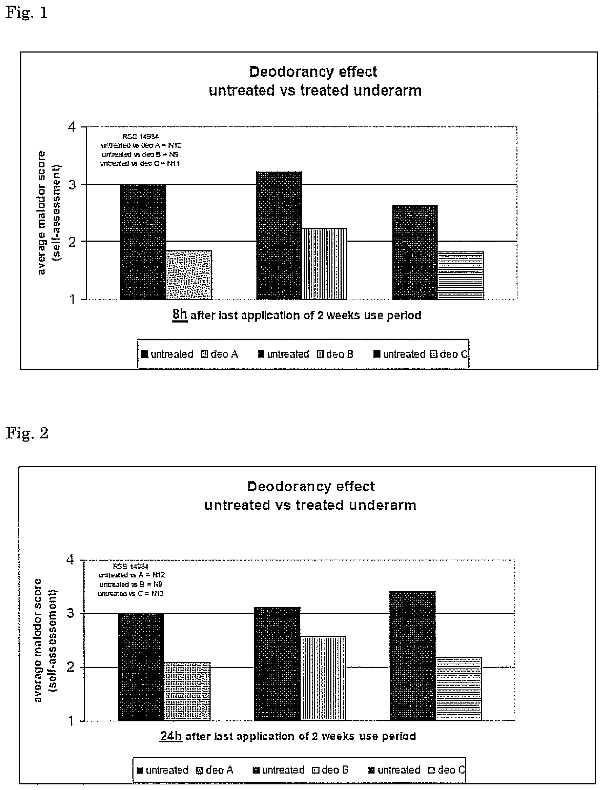

Fig. 1 is a bar diagram reflecting the malodor scores at the 8 hours time point for the three groups, treated vs. untreated.

Fig. 2 is a bar diagram reflecting the malodor scores at the 24 hours time point for the same three groups.

In the figures, the black bars indicated the scores for the untreated armpits. The dotted bars show the scores for composition A, the vertically striped bars show the scores for composition B, and the horizontally striped bars show the scores for composition C.

The test results show that all compositions are active in combating malodor. Further, in terms of absolute ratings, the compositions A and C show the lowest malodor scores after both 8 hours and 24 hours. From the scores relative to the corresponding scores when untreated, composition C shows the longest duration of deodorizing action.

**Claims**

1. The use of a composition as a deodorant on human skin in the presence of sweat, the composition comprising dipropylene glycol as a carrier for a deodorant active agent, wherein the deodorant active agent is selected from the group of consisting of 2-hexyl decanol, 2 octyl dodecanol, and combinations thereof, wherein the carrier and the deodorant active agent are present in an aerosol formulation comprising a propellant.

2. A use according to claim 1, wherein the propellant is a gas selected from propane, isobutane, n-butane and dimethyl ether.

3. A use according to claim 2 wherein the amount of dipropylene glycol is 20 wt.%, the deodorant active agent is 2-octyl dodecanol in an amount of 20 wt.%, and the propellant is isobutane in an amount of 60 wt.%.

4. A use according to claim 2 wherein the amount of dipropylene glycol is 20 wt.%, the deodorant active agent is a 2-hexyl decanol in an amount of 20 wt.%, and the propellant is isobutane in an amount of 60 wt.%.

5. A use according to claim 2 wherein the amount of dipropylene glycol is 20 wt.%, the deodorant active agent is a combination of 2-hexyl decanol in an amount of 16 wt.% and 2-butyl octanol in an amount of 4 wt.%, and the propellant is isobutane in an amount of 60 wt.%.

**Patentansprüche**

1. Verwendung einer Zusammensetzung als Deodorant für menschliche Haut in der Gegenwart von Schweiß, wobei die Zusammensetzung Dipropylenglycol als ein Träger für einen aktiven Deodorant-Wirkstoff umfasst, wobei der aktive Deodorant Wirkstoff aus der Gruppe bestehend aus 2-Hexyldecanol, 2-Octyldodecanol, und Kombinationen davon ausgewählt ist, wobei der Träger und der aktive Deodorant Wirkstoff in einer Aerosol-Formulierung, die ein Treibmittel umfasst, vorliegen.

2. Verwendung nach Anspruch 1, wobei das Treibmittel ein aus Propan, Isobutan, n-Butan und Dimethylether ausgewähltes Gas ist.

3. Verwendung nach Anspruch 2, wobei die Menge an Dipropylenglycol 20 Gew.% beträgt, wobei der aktive Deodorant-Wirkstoff 2-Octyl Dodecanol in einer Menge von 20 Gew.% ist, und wobei der Treibstoff Isobutan in einer Menge von 60 Gew.% ist.

4. Verwendung nach Anspruch 2, wobei die Menge an Dipropylenglycol 20 Gew.% beträgt, wobei der aktive Deodorant-Wirkstoff 2-Hexyldecanol in einer Menge von 20 Gew.% ist, und wobei der Treibstoff Isobutan in einer Menge von 60 Gew.% ist.

5. Verwendung nach Anspruch 2, wobei die Menge an Dipropylenglycol 20 Gew.% beträgt, wobei der aktive Deodorant-Wirkstoff eine Kombination von 2-Hexyldecanol in einer Menge von 16 Gew.% und 2-Butyloctanol in einer Menge von 4 Gew.% ist, und wobei der Treibstoff Isobutan in einer Menge von 60 Gew.% ist.

**Revendications**

1. Utilisation d'une composition en tant que déodorant sur la peau humaine en présence de sueur, la composition comprenant du dipropylène glycol en tant que support pour un agent actif déodorant,
   dans laquelle l'agent actif déodorant est choisi dans le groupe constitué par le 2-hexyl décanol, le 2 octyl dodécanol et leurs combinaisons, dans laquelle le support et l'agent déodorant sont présents dans une formulation aérosol comprenant un agent propulseur.

2. Utilisation selon la revendication 1, dans laquelle l'agent propulseur est un gaz choisi parmi le propane, l'isobutane, le n-butane et l'éther diméthylique.

3. Utilisation selon la revendication 2, dans laquelle la quantité de dipropylène glycol est de 20 % en poids, l'agent actif déodorant est le 2-octyl dodécanol en une quantité de 20 % en poids, et l'agent propulseur est l'isobutane en une quantité de 60 % en poids.

4. Utilisation selon la revendication 2, dans laquelle la quantité de dipropylène glycol est de 20 % en poids, l'agent actif déodorant est un 2-hexyl décanol en une quantité de 20 % en poids, et l'agent propulseur est l'isobutane en une quantité de 60 % en poids.

5. Utilisation selon la revendication 2, dans laquelle la quantité de dipropylène glycol est de 20 % en poids, l'agent actif déodorant est une combinaison de 2-hexyl décanol en une quantité de 16 % en poids et de 2-butyl octanol en une quantité de 4 % en poids, et l'agent propulseur est l'isobutane en une quantité de 60 % en poids.

Fig. 1

**Deodorancy effect**
**untreated vs treated underarm**

8h after last application of 2 weeks use period

Fig. 2

**Deodorancy effect**
**untreated vs treated underarm**

24h after last application of 2 weeks use period

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 295071 A
- EP 1604642 A
- WO 200907089 A
- WO 2009046008 A
- DE 2531343 A2
- DE 10137901 A1
- WO 2008114189 A2
- DE 1961152 A1
- WO 9113606 A2
- US 2009317345 A1
- WO 0185130 A1

**Non-patent literature cited in the description**

- **A. KLUGE et al.** Eigenschaften und Verwendung eines neuen C16-Alkohols. *Seifen - Ole - Fette - Wachse,* 1971, vol. 97 (22), 827-831